(19) Europäisches Patentamt

European Patent Office

Office européen des brevets

(11) EP 0 974 303 B2

(12) **NEW EUROPEAN PATENT SPECIFICATION**

(45) Date of publication and mention
of the opposition decision:
**19.07.2006 Bulletin 2006/29**

(45) Mention of the grant of the patent:
**27.11.2002 Bulletin 2002/48**

(21) Application number: **99114514.5**

(22) Date of filing: **23.07.1999**

(51) Int Cl.:
***A61B 5/00*** (2006.01)

(54) **Method and instrument for measuring blood sugar level**

Verfahren und Vorrrichtung zur Messung des Blutzuckerspiegels

Procédé et appareil de mesure de taux de glucose

(84) Designated Contracting States:
**AT BE CH CY DE DK ES FI FR GB GR IE IT LI LU MC NL PT SE**

(30) Priority: **24.07.1998 JP 20999798**

(43) Date of publication of application:
**26.01.2000 Bulletin 2000/04**

(73) Proprietor: **TERUMO KABUSHIKI KAISHA Tokyo (JP)**

(72) Inventors:
• **Nagashimada, Masaru,**
**c/o Terumo Kabushiki Kaisha**
**Nakakoma-gun,**
**Yamanashi-ken (JP)**
• **Ohyu, Syozo,**
**c/o Terumo Kabushiki Kaisha**
**Nakakoma-gun,**
**Yamanashi-ken (JP)**

(74) Representative: **TBK-Patent**
**Bavariaring 4-6**
**80336 München (DE)**

(56) References cited:
**EP-A- 0 199 484        EP-A- 0 821 234**
**WO-A-88/00812          US-A- 5 114 350**
**US-A- 5 304 468        US-A- 5 885 839**

• **Specification of "Glucotrend"; as well as annexes for demonstrating the introduction of "Glucotrend" on the market**
• **Manual of "One Touch" from the firm Lifescan**

EP 0 974 303 B2

**Description**

BACKGROUND OF THE INVENTION

The present invention relates to a method of measuring blood sugar level for measuring the glucose concentration in blood, and an instrument therefor.

Heretofore, there has been known a blood sugar measuring instrument for measuring the blood sugar level of the blood of a subject by applying blood collected from a finger of the subject to a reagent pad, and by detecting change in color of the reagent pad. In such a blood sugar measuring instrument, light is radiated to the reagent pad, the intensity of reflected light from the reagent pad is measured, and the blood sugar level of the blood applied to the reagent pad is determined.

Change in color of such a reagent pad depends upon the length of time elapsed from the time when the blood was applied to the reagent pad. In the normal practice, therefore, the length of time until the reagent of the reagent pad reacts with glucose in blood is empirically determined, and after the empirically determined time (predetermined time) has elapsed after a subject or a user had instructed the start of measurement, the above-described intensity of reflected light is detected, and the blood sugar level is determined.

In such a method, however, there is dispersion in the length of time after allowing the subject to apply blood to the reagent pad until depressing the switch to instruct the start of measurement. Therefore, even if measurement is started after the predetermined time has elapsed after the start of measurement had been instructed, the result of measurement may include error. Also, if measurement was started after the predetermined time had elapsed considering the delay in time after applying blood to the reagent pad until instructing the start of measurement without using the above-described switch, there was a problem in which the subject or the user always had to wait for longer time resulting in increase in time required for measurement.

A conventional method and instrument for calculating the blood sugar level after a fixed predetermined time period is disclosed in the WO-A-88/00812.

Further, it is referred to the US-A-5,304,468 according to which the blood sugar level is calculated by using a mathematical expression for fitting.

SUMMARY OF THE INVENTION

An object of the invention is to provide a method and an instrument for measuring the blood sugar level of blood which can measure the blood sugar level accurately by automatically determining an appropriate timing for the measurement.

More specifically, it is an object of the invention to provide a method and an instrument for measuring the blood sugar level which can determine the timing for starting the measurement automatically corresponding to a change in the color of a reagent pad or specimen when blood is applied thereto.

Still another object is to provide a method and an instrument for measuring the blood sugar level which plainly inform the subject or the user of the process state of the blood sugar level measurement.

In order to attain the above objects, there is provided a blood sugar measuring instrument according to claim 1 and a method for measuring the blood sugar level of blood according to claim 9.

Other features and advantages of the present invention will be apparent from the following descriptions taken in conjunction with the accompanying drawings, in which like reference characters designate the same or similar parts throughout the figures thereof.

BRIEF DESCRIPTION OF THE DRAWINGS

The accompanying drawings, which are incorporated in and constitute a part of the specification, illustrate embodiments of the invention and, together with the descriptions, serve to explain the principle of the invention.

Fig. 1 is a block diagram showing the constitution of a blood sugar measuring instrument of an embodiment of the present invention;
Fig. 2 is a schematic diagram showing the blood sugar measuring instrument of the embodiment of the present invention;
Figs. 3A and 3B are graphs illustrating the quantity of reflected light and absorbency in the blood sugar measuring instrument of an embodiment of the present invention;
Fig. 4 is a diagram illustrating a method for determining the end point of the measurement by the blood sugar measuring instrument of the embodiment of the present invention;
Fig. 5 is a flowchart showing the process of measurement by the blood sugar measuring instrument of the embodiment of the present invention;

Fig. 6 is a diagram illustrating change in absorbency by blood sugar levels;

Fig. 7 is a diagram showing relationship between blood sugar levels and measuring time;

Fig. 8 is a diagram showing the display mode in a blood sugar measuring instrument of the second embodiment of the present invention;

Fig. 9 is a diagram illustrating a method for determining the end point of the measurement by the blood sugar measuring instrument of the second embodiment of the present invention;

Fig. 10 is a diagram showing time since the display mode has been changed until the end of measurement corresponding to blood sugar levels in the second embodiment of the present invention;

Fig. 11 is a flowchart showing the process of measurement by the blood sugar measuring instrument of the second embodiment of the present invention;

Fig. 12 is a diagram showing change in absorbency of the same blood due to ambient temperatures; and

Fig. 13 is a graph showing change in absorbency due to temperatures.

DETAILED DESCRIPTION OF THE PREFERRED EMBODIMENTS

The preferred embodiments of the present invention will be described in detail below referring to attached drawings.

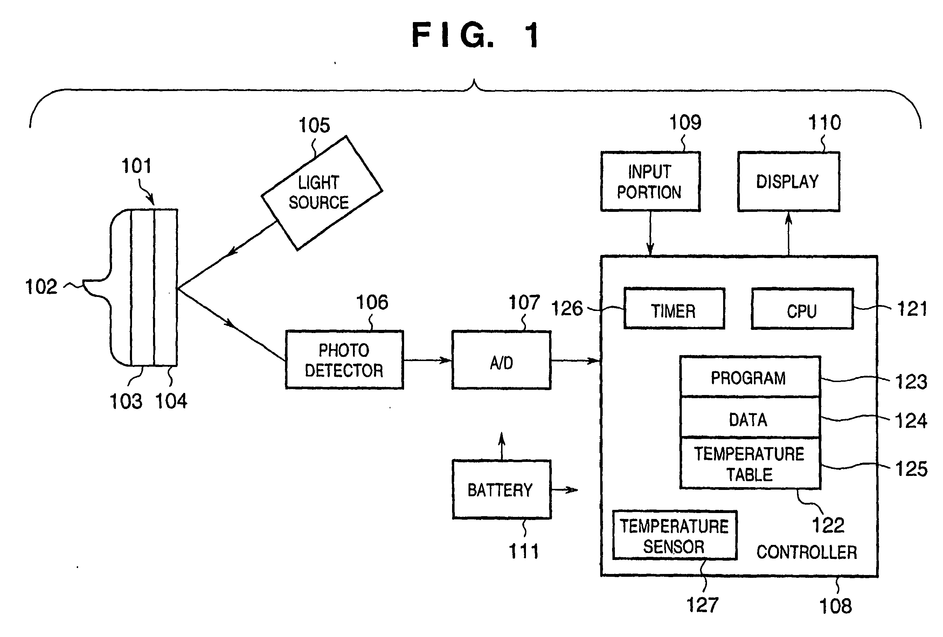

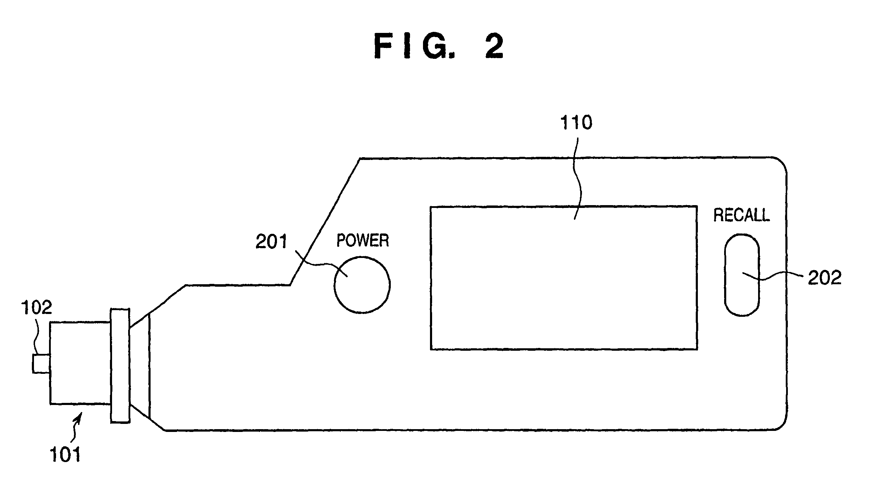

Fig. 1 is a block diagram schematically showing the constitution of a blood sugar measuring instrument of an embodiment of the present invention, and Fig. 2 is a schematic diagram of this blood sugar measuring instrument.

In these drawings, 101 designates a specimen (chip) which is detachably mounted on the front end of the blood sugar measuring instrument as Fig. 2 shows, and 102 designates an opening for collecting blood, which is linked to a reagent layer portion through a blood traveling portion formed of capillaries.

This reagent layer portion comprises a reagent layer 103 containing a reagent, and a blood cell filtering layer 104 for filtering blood cells. This reagent layer 103 is impregnated with a reagent required for reacting with sugar in blood and producing color, such as for example, glucose oxidase (GOD), peroxidase (POD), 4-aminoantipyrine, and N-ethyl-N-(2-hydroxy-3-sulfopropyl)-m-toluidine-sodium (TOOS). The pore diameter of the film forming the reagent layer 103 is preferably a diameter allowing blood cells to pass through, for example, 5-15 $\mu$m. By forming such a diameter, the development of blood into the reagent layer 103 is expedited, thereby speed of reaction of blood with the reagent can be increased. As the material for the film, conventionally known materials, such as nitrocellulose, can be used. The blood cell filtering layer 104 is constituted by a film having a pore diameter for filtering blood cells, for example, 0.45 $\mu$m. As the material for the film, conventionally known materials, such as polyethersulfone, can be used.

Blood enters inside the chip from the opening 102, travels through the traveling portion comprising capillaries, and reaches the reagent layer portion, where blood reacts with the reagent contained in the reagent layer 103, and is colored. Blood further travels to the blood cell filtering layer 104, where the blood cells are filtered. Reflected light is then measured from the blood cell filtering layer side.

The reagent layer portion may be constituted by one layer of film having fine pores allowed blood cell filtering and impregnated with the reagent, or may have a conventionally known multilayer structure.

A light source 105 generates light having a predetermined wavelength, for example, 610 nm. A photo detector 106 detects the intensity of light irradiated by the light source 105 reflected by the blood cell filtering layer 104. By this intensity of reflected light, change in color in the blood cell filtering layer 104 can be detected. An A/D converter 107 converts detection signals (analog signals) outputted from the photo detector 106 corresponding to the intensity of reflected light into digital signals.

A controller 108 controls the operation of the entire instrument, and comprises, for example, a CPU 121 such as a microprocessor, a program memory 123 for storing the control program of the CPU 121 and the like, a data memory 124 which is the RAM area for storing measured data and the like, and a temperature table 122 for storing correction data corresponding to ambient temperature (environmental temperature) sensed by a temperature sensor 127 and the like. A timer 126 measures the lapse of time as described later, and informs it to the CPU 121 by interruption or the like.

An input portion 109 comprises a key (201 in Fig. 2) for instructing the power source to be turned on or off, a key (202 in Fig. 2) for instructing the reading of measured data or the like. Numeral 110 is a display such as a liquid crystal display, and 111 is a battery for supplying electric power to the entire instrument.

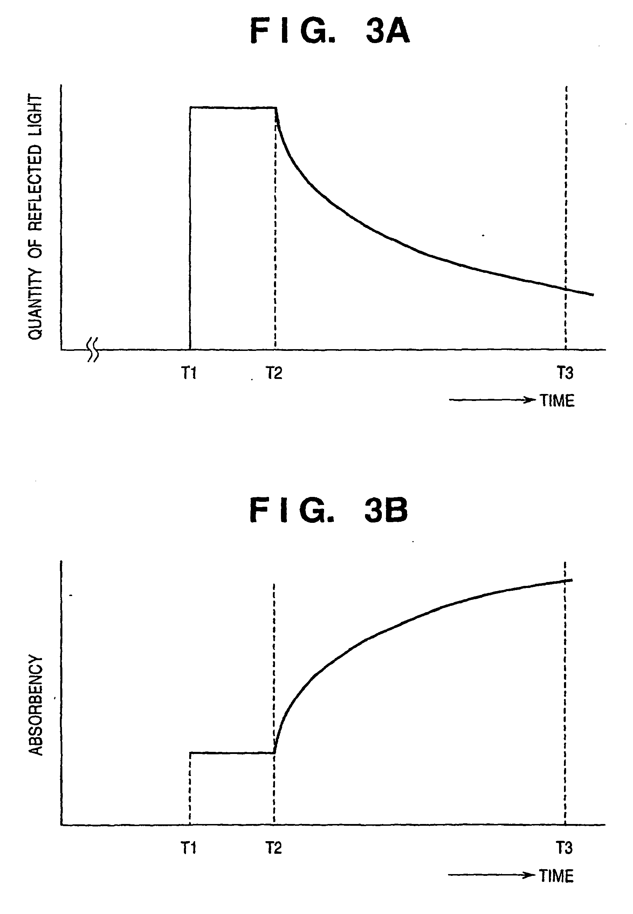

Fig. 3A is a graph showing an example of the quantity of detected reflected light; Fig. 3B is a graph showing absorbency corresponding to the intensity of the reflected light (Fig. 3A).

In Figs. 3A and 3B, timing T1 shows the time when the chip 101 is mounted on the instrument. That is, since the reflecting surface of the blood cell filtering layer 104 of the chip 101 has a nearly white color initially (before coloration), when the chip 101 is mounted on the instrument at timing T1, the quantity of reflected light increases rapidly. In timing T1-T2, blood applied to the opening 102 travels through the traveling portion, reaches the reagent layer 103, and reacts with the reagent to produce color. Thereafter, the colored blood travels to the blood cell filtering layer 104 to color the reflecting surface, thereby the quantity of reflected light gradually begins lowering after the timing T2. When the reaction of the reagent with blood has almost completed (timing T3), the blood sugar level is calculated. The method of determining this timing T3 will be described later.

Absorbency shown in Fig. 3B is given from the quantity of reflected light before the coloration of the reagent layer takes place (Aini) and the quantity of reflected light after the coloration of the reagent layer takes place (Aaft) as:

$$Absorbency = (Aini)/(Aaft)$$

The above-described measuring timing T3 is given as the time when this absorbency has reached almost equilibruim.

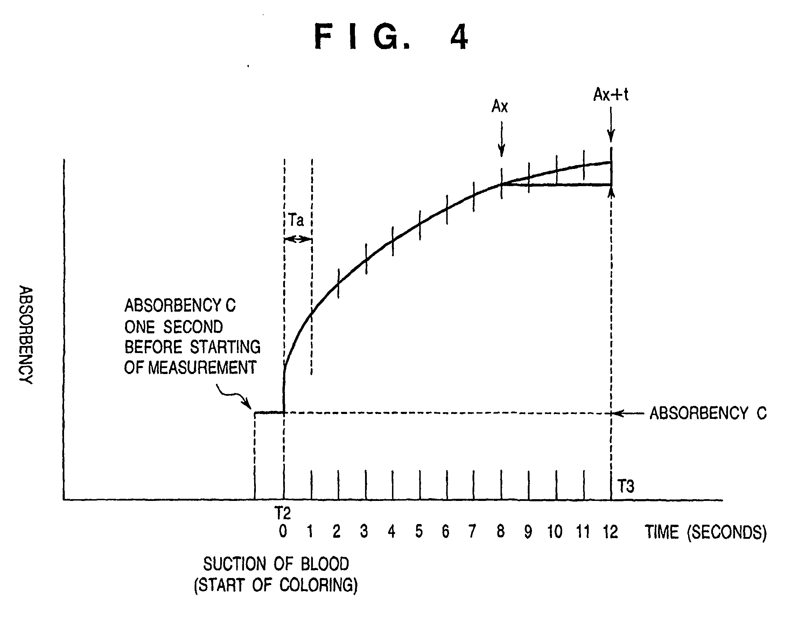

Fig. 4 is a diagram illustrating how to determine the measuring timing T3 in this embodiment. Although the measuring timing T3 is shown about 12 seconds after starting the coloration of the reagent layer 104 in this example, this is of course only an example.

Absorbency one second before starting coloration at timing T2 is represented by C, and thereafter, the intensity of reflected light is measured at the time interval of Ta seconds (e.g. 1 second) to calculate absorbency. If absorbency X seconds afterstarting measurement is represented by Ax, and absorbency (X +t) seconds afterstarting measurement is represented by Ax+t ($X \geq t$: e.g. t = 4 sec):

$$\Delta Ax = Ax - C$$

$$\Delta Ax{+}t = Ax{+}t - C$$

$$(\Delta Ax{+}t - \Delta Ax)/t = \Delta D \text{ (change in absorbency per second:}$$

$$\text{averaged value in t seconds)}$$

$$(\Delta D/\Delta Ax{+}t) \times 100 \leq d\ (\%) \qquad (d = 2\ (\%))$$

That is, on the basis of absorbency X seconds after starting measurement, Ax, and absorbency (X+t) seconds after starting measurement. Ax+t, the reaction of the reagent with the blood is considered to have almost completed when change in absorbency per second, $\Delta D$, has become d (2%) or less, and the blood sugar level is determined on the basis of absorbency at that time.

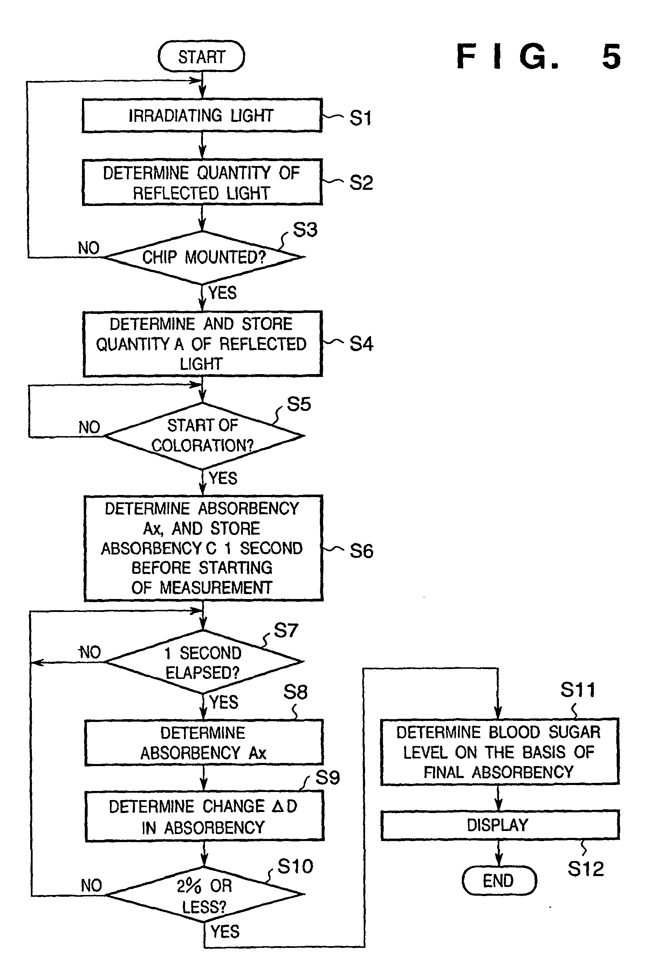

Fig. 5 is a flowchart showing the process of measurement in the blood sugar measuring instrument of this embodiment. The control program for implementing this process is stored in the program memory 123 of the above-described controller 108.

First, in step S1, the light source 105 is-made to emit light intermittently, and in step S2, the intensity of reflected light detected by the photo detector 106 is read. Whether the chip 101 is mounted or not is detected on the basis of the intensity of the reflected light (step S3), and when the intensity of the reflected light increases as shown in the timing T1 of Fig.- 3A, it is judged that the chip 101 has been mounted.

The process thus proceeds from step S3 to step S4, when the quantity of reflected light A is obtained and stored in the data memory 124. Next, in step S5, whether the reagent layer 104 has started coloration or not is checked. When the coloration of the reagent layer 104 has started and the quantity of reflected light has started lowering, the process proceeds to step S6, and the time is judged to be the timing T2 for starting measurement. Absorbency one second before T2, C, is obtained and stored in the data memory 124 with the intensity of the reflected light at that time, and absorbency Ax is determined on the basis of the intensity of reflected light at the start of the measurement. Thus, the process is proceeded to the measurement of blood sugar shown by timings T2 to T3 in Figs. 3A and 3B.

In this measurement, first in step S7, elapsing one second is waited on the basis of time measurement with the timer 126, and when one second is elapsed, the process is proceeded to step S8 to obtain absorbency Ax on the basis of the intensity of the reflected light at that time. The process is then proceeded to step S9, where whether absorbency t seconds before, Ax-t, is stored or not is checked, if stored, then the difference (Ax - Ax-t) is obtained, and change in absorbency per second ($\Delta D$) is determined from the averaged value. The process is proceeded to step S10, where whether the value of the change (ΔD) is 2% or less is checked, and if not, the process is returned to step S7 and the above-described process is repeated. In first 4 seconds from the start of measurement, since no data on previous absorbency are present, the process of step S9 is practically skipped.

Thus, in step S10, the process is proceeded to step S11 when change in absorbency per second becomes 2% or less, and on the basis of absorbency at that time, the concentration of glucose contained in the blood applied to the chip 101 is calculated to determine the blood sugar level of the subject. The result is indicated on the display 110. The formula for calculating the concentration of glucose X is represented, for example, when the absorbency at the time of measurement is K, as follows:

$$X = a0 + a1 \cdot K + a2 \cdot K^2 + a3 \cdot K^3$$

where a0, a1, a2, and a3 are constants.

The value d for determining time intervals for measurement Ta and t, and timing for measurement T3 in the above-described embodiment is not limited to the above values, but it is of course possible to store the relationship between absorbency experimentally obtained and the concentration of glucose as a table, and to determine the corresponding blood sugar level from finally obtained absorbency.

In this embodiment, since the speed of reaction of the reagent in the reagent layer 103 varies depending on environmental temperatures, the measured absorbency or the finally determined blood sugar level is corrected referring to the temperature table 122 on the basis of the temperature value measured by the temperature sensor 127.

According to this embodiment, it is not required to always wait a predetermined time until the result of measurement is obtained, and since the measurement is performed at the most effective timing (the time when the reaction of the reagent has almost completed), accurate results of measurement can be obtained.

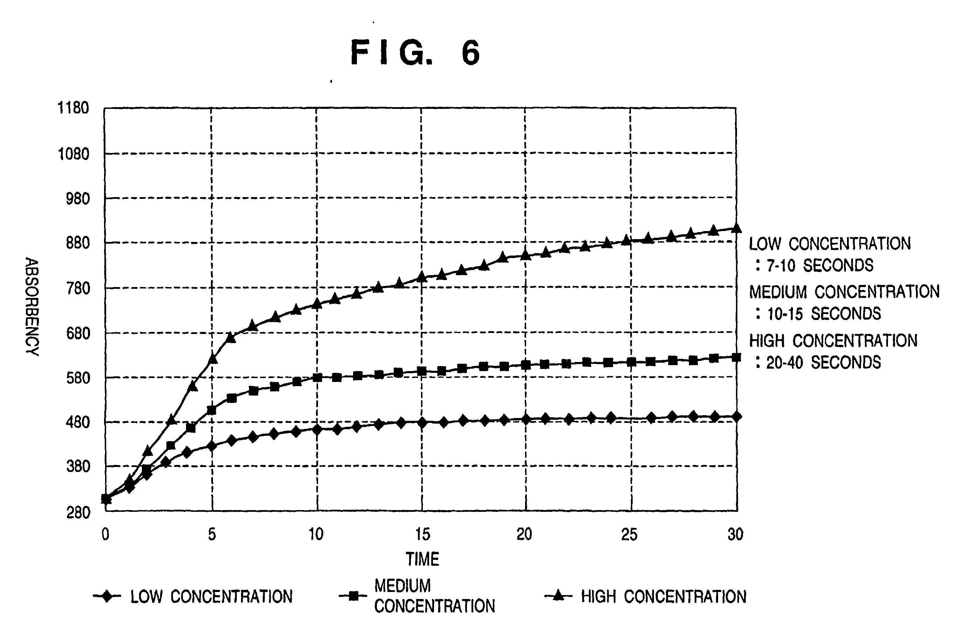

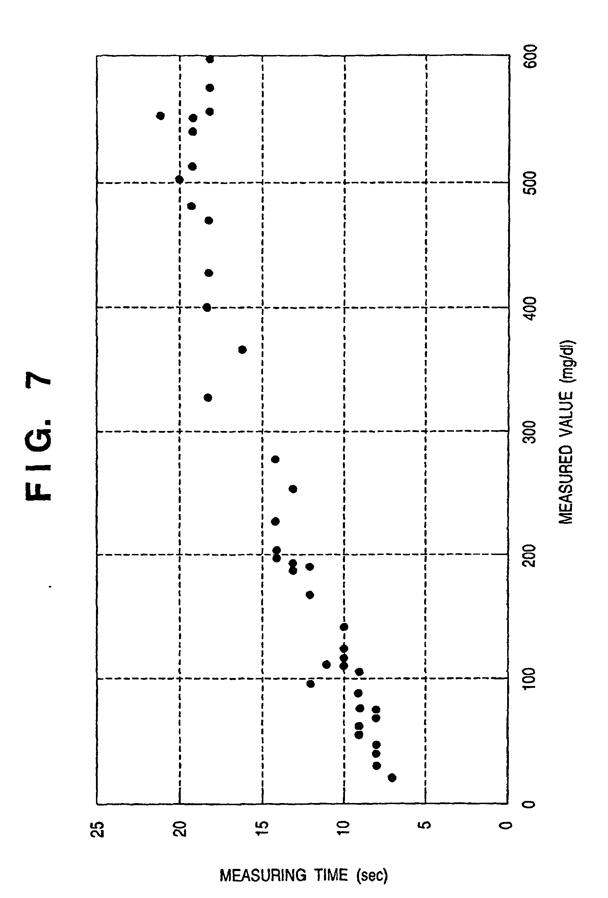

Fig. 6 is a graph showing the relationship between the concentration of glucose in blood and absorbency and Fig. 7 is a graph showing the relationship between the concentration of glucose in blood and the length of time required for measurement.

As apparent from these graphs, it is known that the time until the absorbency almost reaches equilibrium increases with increase in the concentration of glucose. That is, time required for measurement decreases for blood having a low glucose concentration, and the time increases with increase in the concentration. Therefore, for example, an accurate blood sugar level can be measured even if the concentration of glucose is high.

[Embodiment 2]

Next, Embodiment 2 of the present invention will be described. In this Embodiment 2, an instrument and method for displaying the state of measurement, especially effective when the length of time until measurement is completed is not constant as above-described Embodiment 1.

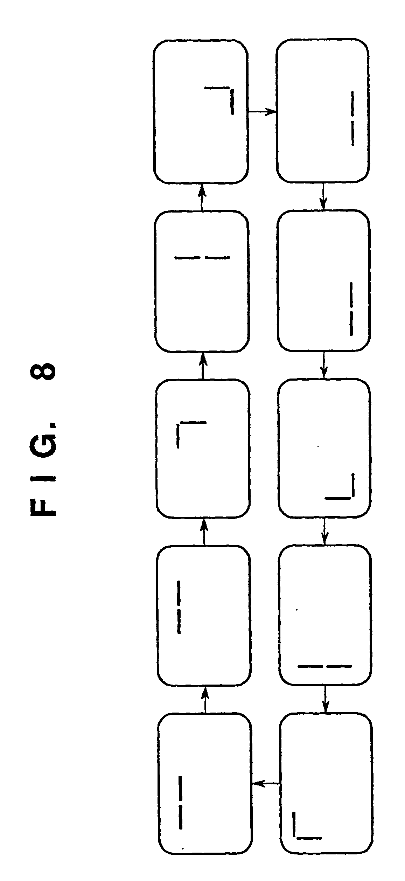

In this Embodiment 2, rotating and moving marks, for example, as shown in Fig. 8 are displayed on the display 110 (roulette display), and the speed of rotating and moving is changed corresponding to the remaining time until the completion of measurement. In Fig. 8, the modes of displaying change in the direction shown by arrows.

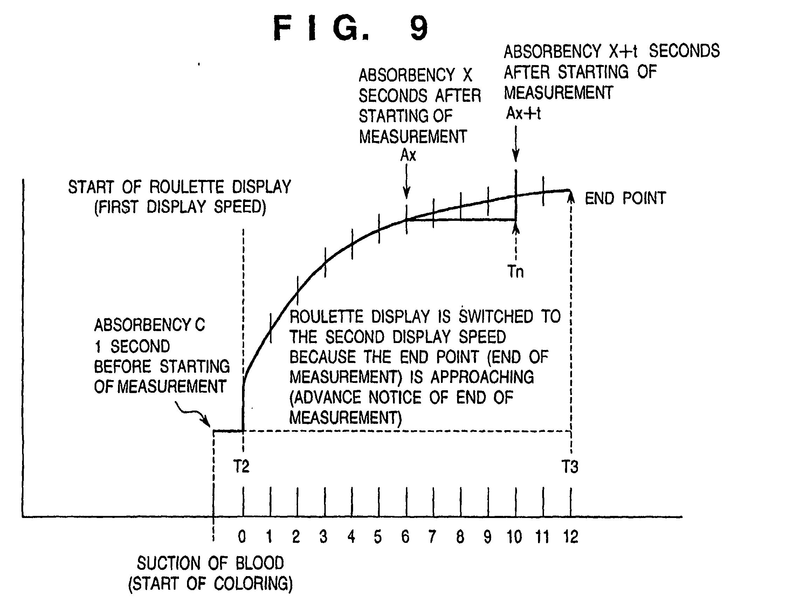

Fig. 9 is a diagram illustrating such a method of displaying, and corresponds to the above-described Fig. 4.

As in case of Fig. 4, absorbency when coloration begins at timing T2 is represented by C, and thereafter, the intensity of reflected light is measured at the time interval of Ta seconds (e.g. 1 second) to calculate absorbency. At the same time, at timing T2, marks, for example shown in Fig. 8, are displayed, and the modes of displaying are shifted in the direction of arrows in Fig. 8, for example, each time one second is elapsed (the first display mode).

If absorbency X seconds after starting measurement is represented by Ax, and absorbency (X + t) seconds after starting measurement is represented by Ax+t (X ≥ t: e.g. t = 4 sec):

$$\Delta Ax = Ax - C$$

$$\Delta Ax{+}t = Ax{+}t - C$$

$$(\Delta Ax+t - \Delta Ax)/t = \Delta D \text{ (change in absorbency per second:}$$

$$\text{averaged value in t seconds)}$$

$$(\Delta D/\Delta Ax+t) \times 100 \leq d1 \; (\%) \qquad (d1 = 5 \; (\%))$$

That is, on the basis of absorbency X seconds after starting measurement, Ax, and absorbency (X + t) seconds after starting measurement, Ax+t, the moving speed of the marks displayed on the display 110 is changed when change in absorbency per second, $\Delta D$, has become d (5%) or less (the second display mode: e.g. about every one second) to inform the user or the subject that the completion of measurement is approached. Finally, when change in absorbency per second has become 2% or less, it is judged that the reaction of the reagent with the blood has almost completed, and the blood sugar level is determined on the basis of absorbency at that time.

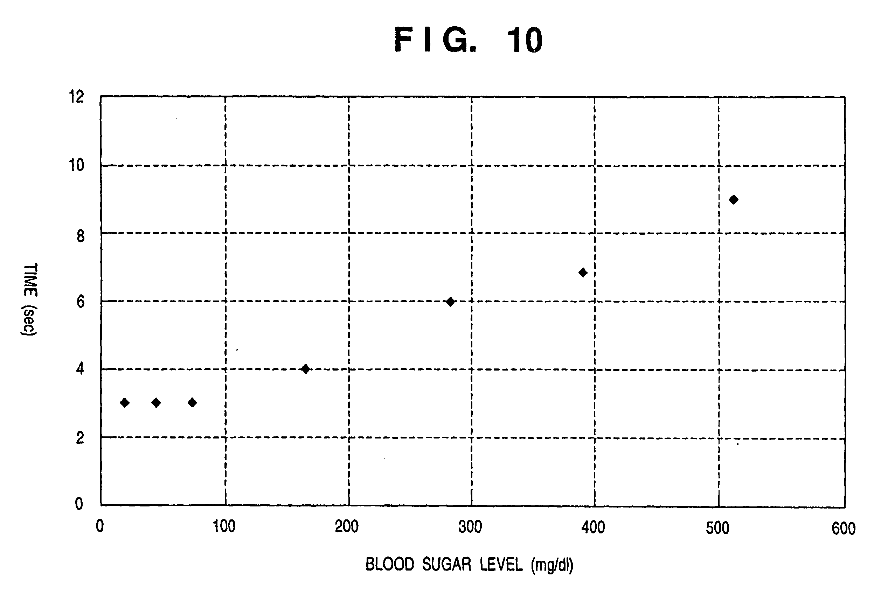

Fig. 10 is a diagram showing time T from the time when the display is shifted to the above-described second mode to the time when the measurement is completed corresponding to blood sugar levels (glucose concentrations).

In this diagram also, it is known that the length of time required for measurement increases with increase in blood sugar levels. For example, when the glucose concentration is low, the above-described time T is about 3 seconds; when the concentration is medium, T is about 3 to 6 seconds; and when the concentration is high, T is about 6 to 9 seconds.

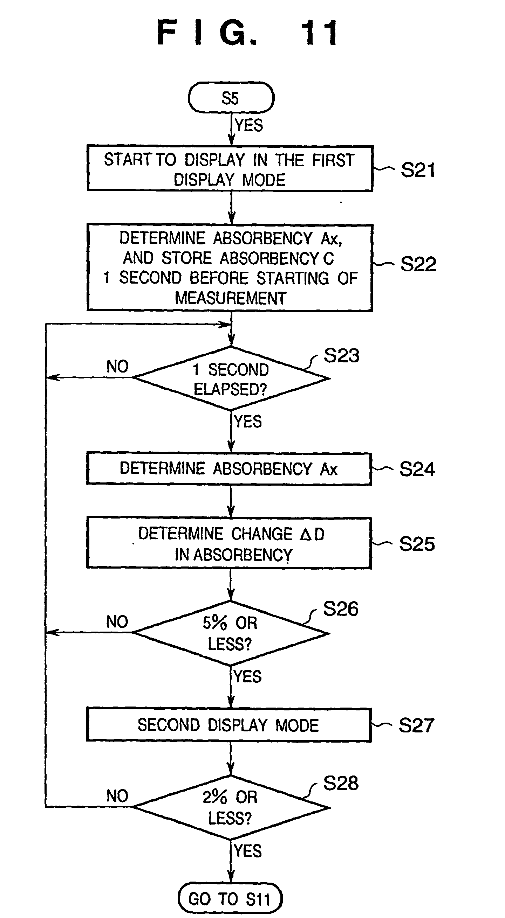

Fig. 11 is a flowchart showing the process of measurement in this Embodiment 2, from which description of the portions same as those of the above-described Fig. 5 are omitted.

Here, when the coloration of the blood cell filtering layer 104 in the above-described Fig. 5 begins, the process is proceeded to step S21, and display in the first display mode is started. Then, the process is proceeded to step S22, the time is judged as the timing for starting measurement, T2, and absorbency one second before T2, C, is determined and stored in the data memory 124 with the intensity of the reflected light at that time, and absorbency Ax is determined on the basis of the intensity of reflected light at that time. Then, the process is proceeded to blood sugar measuring process shown by timings T2 and T3 in Fig. 9.

In this measurement, first in step S23, elapsing one second is waited on the basis of time measurement with the timer 126, and when one second is elapsed, the process is proceeded to step S24 to obtain absorbency Ax on the basis of the intensity of the reflected light at that time. The process is then proceeded to step S25, where whether absorbency t seconds before, Ax-t, is stored or not is checked, if stored, then the difference (Ax - Ax-t) is obtained, and change in absorbency per second ($\Delta D$) is determined from the averaged value. The process is proceeded to step S26, where whether the value of the change ($\Delta D$) is 5% or less is checked, and if not, the process is returned to step S23 and the above-described process is repeated. In first 4 seconds from the start of the measurement, since no data on previous absorbency are present, the process of the step S25 is practically skipped. When change per second has become 5% or less, the process is proceeded to step S27, and the display mode is shifted to the second display mode in which the moving speed of the above-described marks is changed (e.g. lowered). Furthermore, in the next step S27, whether the value of change $\Delta D$ is 2% or less is checked, and if not, the process is returned to step S23, but when change per second has become 2% or less, the process is proceeded to step S11 in Fig. 5 described above, and on the basis of absorbency at that time, the concentration of glucose contained in the blood applied to the chip 101 is calculated to determine the blood sugar level of the subject. The result is indicated on the display 110.

The above-described first and second display modes are not limited to the display of this embodiment, but may be displays such as digital displays or clocks.

When the modes of displaying is changed from the first display mode to the second display mode, it is preferable that the length of time from shifting to the second display mode to the completion of measurement is substantially constant, because the user can estimate the length of time until the completion of measurement. In this case, since the time taken for measurement differs depending on the blood sugar level as described above, timing for shifting from the first display mode to the second display mode is changed depending on absorbency.

That is, as the above-described Fig. 6 shows, when absorbency is a certain value (e.g. 600) or more, the condition to shift to the second display mode in the above-described Fig. 9 (change per second $\Delta D$: 3%) is established. By this, in the case of high concentration, timing to shift to the second display mode is delayed, and even if the time until the completion of measurement increases due to a high blood sugar level, the length of time after shifting to the second display mode to the completion of measurement can be substantially constant.

Also in the above-described Embodiments 1 and 2, the temperature table 122 stores correction information at, for example, 10°C, 15°C, 20°C, and 30°C, and on the basis of this correction information, the obtained blood sugar levels are corrected.

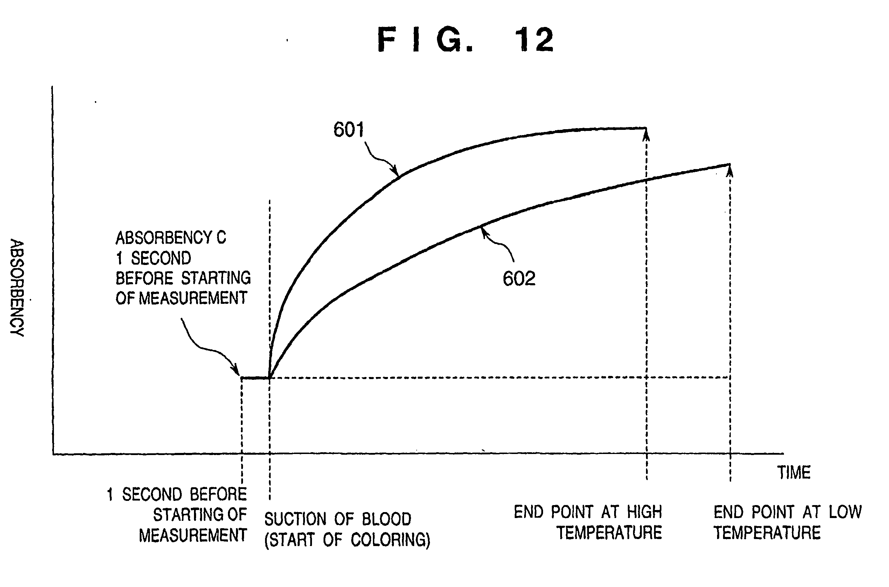

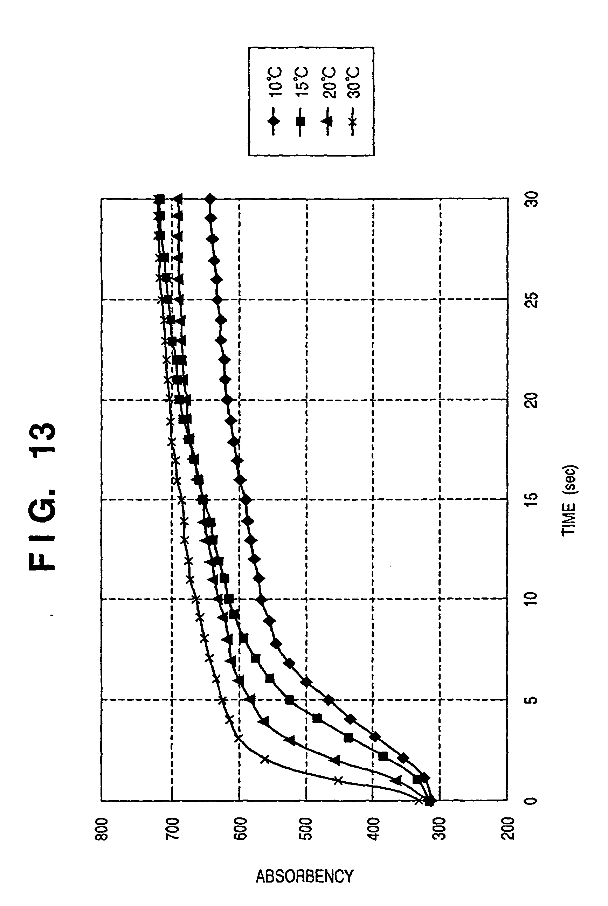

Fig. 12 is a diagram showing change in absorbency of the same blood depending on ambient temperatures, and Fig. 13 is a graph showing difference in absorbency due to temperatures.

In Fig. 12, numeral 601 denotes change in absorbency when ambient temperature is high, and 602 denotes change in absorbency when ambient temperature is low. Thus, the coloration of the reagent is delayed as ambient temperature lowers, the time until the end point is reached becomes long, and the absorbency at the completion of measurement is also lowered. On the contrary, as ambient temperature increases, the coloration of the reagent becomes quick, the time until the end point is reached becomes short, and the absorbency at the completion of measurement is also increased.

Therefore, in the blood sugar measuring instrument of this embodiment, in order to correct such a measurement error of the blood sugar level due to ambient temperature, a temperature sensor 127 is provided for sensing ambient temperature, and conversion from the absorbency to the blood sugar level for obtaining the blood sugar level from the absorbency is corrected.

As described above, according to this embodiment, display can be performed at most adequate timing.

Also, according to this embodiment, since the state of measurement can be informed to the user, there is the effect of ease of using.

Also, the situation in which the user must unnecessarily wait until the completion of measurement can be prevented.

Also, the occurrence of measurement error due to temperature can be prevented.

**Claims**

1.  A blood sugar measuring instrument for measuring the blood sugar level of blood on the basis of change in the color of a specimen (101) in which a reagent has reacted with the blood, said instrument comprising:

    reflected light detecting means (105, 106) for irradiating light to said specimen (101) at time intervals (Ta) and for detecting the intensity of the reflected light from the specimen (101);
    change detecting means for detecting a change in quantity of reflected light ($\Delta D/\Delta Ax+t$) at said time intervals (Ta); and
    blood sugar calculating means for calculating the blood sugar level of said blood on the basis of a quantity of reflected light ($\Delta Ax+t$) corresponding to the latest intensity of the reflected light detected by said reflected light detecting means (105, 106) when the change in quantity of reflected light ($\Delta D/\Delta Ax+t$) detected by said change detecting means has become a first predetermined quantity (d) or less;

    **characterized in that**
    said change detecting means obtains a difference ($\Delta Ax+t - \Delta Ax$) between the quantity of reflected light ($\Delta Ax+t$) corresponding to the latest intensity of the reflected light detected by said reflected light detecting means (105, 106) and a quantity ($\Delta Ax$) of reflected light corresponding to the intensity of the reflected light detected by said reflected light detecting means before a predetermined period of time (t) which is longer than the time intervals (Ta), and determines the change in quantity of reflected light ($\Delta D/\Delta Ax+t$) by dividing said difference ($\Delta Ax+t - \Delta Ax$) by the predetermined period of time (t) and the quantity of reflected light ($\Delta Ax+t$) corresponding to the latest intensity of the reflected light detected by said reflected light detecting means (105, 106).

2.  The blood sugar measuring instrument according to claim 1, wherein said change detecting means obtains the quantities of reflected light ($\Delta Ax+t$, $\Delta Ax$) on the basis of absorbency (A) obtained from the intensity ($Aini$) of the reflected light from said specimen (101) before reacting with said blood, and from the intensity ($Aaft$) of the reflected light after said specimen (101) has been colored by the reaction with said blood.

3.  The blood sugar measuring instrument according to claim 1 or 2, further comprising:

    notifying means for notifying that the blood sugar level is being measured; and
    notifying control means for controlling to change the mode of notification of said notifying means when said change in quantity of reflected light ($\Delta D/\Delta Ax+t$) is larger than said first predetermined quantity (d), and becomes a second predetermined quantity (d1) which is present before said first predetermined quantity (d).

4.  The blood sugar measuring instrument according to claim 3, wherein said notifying control means further notifies that measurement is started when the coloration of said specimen is detected.

5.  The blood sugar measuring instrument according to any of claims 1 to 4, wherein

said specimen (101) has an opening (102) for applying blood, a traveling portion to which blood applied to said opening travels, and a reagent layer portion (103, 104) colored by the reaction with blood having passed through said traveling area, and

said reflected light detecting means (105, 106) detects reflected light from said reagent layer portion.

6. The blood sugar measuring instrument according to claim 5, wherein said reagent layer portion comprises a reagent layer (103) which is colored by the reaction with blood, and a filter layer (104) for filtering blood cells of the blood.

7. The blood sugar measuring instrument according to claim 5, wherein the initial color of said reagent layer portion (103, 104) is substantially white.

8. The blood sugar measuring instrument according to claim 3, wherein said notifying means moves and displays a predetermined mark at a predetermined speed, and that said notifying control means changes the speed of movement of said predetermined mark when said second predetermined quantity (d1) is reached.

9. A method for measuring the blood sugar level of blood on the basis of change in the color of a specimen (101) in which a reagent has reacted with the blood, said method comprising the steps of:

detecting reflected light by irradiating light to said specimen (101) at time intervals (Ta) and detecting the intensity of the reflected light from the specimen (101);

detecting a change in quantity of reflected light ($\Delta D/\Delta Ax+t$) at said time intervals (Ta); and

calculating the blood sugar level of said blood on the basis of a quantity of reflected light ($\Delta Ax+t$) corresponding to the latest intensity of the reflected light detected in said step of detecting reflected light when the change in quantity of reflected light ($\Delta D/\Delta Ax+t$) detected in said step of detecting change has become a first predetermined quantity (d) or less,

**characterized in that**

said step of detecting change includes obtaining a difference ($\Delta Ax+t - \Delta Ax$) between the quantity of reflected light ($\Delta Ax+t$) corresponding to the latest intensity of the reflected light detected in said step of detecting reflected light and a quantity ($\Delta Ax$) of reflected light corresponding to the intensity of the reflected light detected in said step of detecting reflected light before a predetermined period of time (t) which is longer than the time intervals (Ta), and determining the change in quantity of reflected light ($\Delta D/\Delta Ax+t$) by dividing said difference ($\Delta Ax+t - \Delta Ax$) by the predetermined period of time (t) and the quantity of reflected light ($\Delta Ax+t$) corresponding to the latest intensity of the reflected light detected by said step of detecting reflected light.

10. The method according to claim 9, wherein in said step of detecting change the quantities of reflected light ($\Delta Ax+t$, $\Delta Ax$) :are obtained on the basis of absorbency (A) obtained from the intensity (Aini) of the reflected light from said specimen (101) before reacting with said blood, and from the intensity (Aaft) of the reflected light after said specimen (101) has been colored by the reaction with said blood.

11. The method for measuring the blood sugar level of blood according to claim 9 or 10, further comprising the steps of:

notifying that the blood sugar level is being measured; and

controlling to change the mode of notification of said notifying step when said change in quantity of reflected light ($\Delta D/\Delta Ax+t$) is larger than said first predetermined quantity (d), and becomes a second predetermined quantity (d1) which is present before said first predetermined quantity (d).

12. The method for measuring the blood sugar level of blood according to claim 11, wherein said notifying control step further notifies that measurement is started when the coloration of said specimen is detected.

13. The method for measuring the blood sugar level of blood according to any of claims 9 to 12, wherein said specimen (101) has an opening (102) for applying blood, a traveling area to which blood applied to said opening travels, and a reagent layer portion (103, 104) colored by the reaction with blood having passed through said traveling area, and

said reflected light detecting step detects reflected light from said reagent layer portion.

14. The method for measuring the blood sugar level of blood according to claim 13, wherein said reagent layer portion comprises a reagent layer (103) which is colored by the reaction with blood, and a filter layer (104) for filtering blood cells of the blood.

15. The method for measuring the blood sugar level of blood according to claim 13, wherein the initial color of said reagent layer portion (103, 104) is substantially white.

16. The method for measuring the blood sugar level of blood according to claim 11, wherein said notifying step moves and displays a predetermined mark at a predetermined speed, and that said notifying control step changes the speed of movement of said predetermined mark when said second predetermined quantity (d1) is reached.

17. The method for measuring the blood sugar level of blood according to claim 11 or 16, wherein said notifying control step changes said second predetermined quantity (d1) corresponding to the measured glucose concentration.

18. The method for measuring the blood sugar level of blood according to any one of claims 9 to 17, wherein said step of calculating the blood sugar level measures ambient temperature, and determines said blood sugar level corresponding to said ambient temperature.


**Patentansprüche**

1. Blutzuckermessinstrument zur Messung des Blutzuckerspiegels von Blut auf Grundlage der Farbänderung einer Probe (101), in der ein Reagens mit dem Blut reagiert hat, wobei das Instrument Folgendes umfasst:

   eine Reflexionslichterfassungseinrichtung (105, 106) zur Lichtabstrahlung auf die Probe (101) in Zeitabständen (Ta) und zum Erfassen der Stärke des Reflexionslichts von der Probe (101);
   eine Änderungserfassungseinrichtung zur Erfassung einer Änderung der Reflexionslichtintensität ($\Delta D/\Delta Ax+t$) in den Zeitabständen (Ta); und
   eine Blutzuckerberechnungseinrichtung zur Berechnung des Blutzuckerspiegels des Bluts auf Grundlage einer Reflexionslichtintensität ($\Delta Ax+t$), die der letzten von der Reflexionslichterfassungseinrichtung (105, 106) erfassten Stärke des Reflexionslichts entspricht, wenn die von der Änderungserfassungseinrichtung erfasste Änderung der Reflexionslichtintensität ($\Delta D/\Delta Ax+t$) kleiner oder gleich einer ersten vorbestimmten Intensität (d) geworden ist,

   **dadurch gekennzeichnet, dass**
   die Änderungserfassungseinrichtung eine Differenz ($\Delta Ax+t - \Delta Ax$) zwischen der Reflexionslichtintensität ($\Delta Ax+t$), die der letzten von der Reflexionslichterfassungseinrichtung (105, 106) erfassten Stärke des reflektierten Lichts entspricht, und einer Reflexionslichtintensität ($\Delta Ax$) ermittelt, die der Intensität des reflektierten Lichts entspricht, die von der Reflexionserfassungseinrichtung eine vorbestimmte Zeitdauer (t) zuvor erfasst wurde, die länger als die Zeitabstände (Ta) ist, und die Änderung der Reflexionslichtintensität ($\Delta D/\Delta Ax+t$) bestimmt, indem sie die Differenz ($\Delta Ax+t - \Delta Ax$) durch die vorbestimmte Zeitdauer (t) und die Reflexionslichtintensität ($\Delta Ax+t$) teilt, die der letzten von der Reflexionslichterfassungseinrichtung (105, 106) erfassten Stärke des Reflexionslichts entspricht.

2. Blutzuckermessinstrument nach Anspruch 1, wobei die Änderungserfassungseinrichtung die Reflexionslichtintensitäten ($\Delta Ax+t$, $\Delta Ax$) auf Grundlage des Absorptionsgrads (A) ermittelt, der anhand der Stärke (Aini) des von der Probe (101) stammenden Reflexionslichts vor der Reaktion mit dem Blut und anhand der Stärke (Aaft) des Reflexionslichts nach der Färbung der Probe durch die Reaktion mit dem Blut ermittelt wird.

3. Blutzuckermessinstrument nach Anspruch 1 oder 2, mit außerdem:

   einer Meldeeinrichtung zum Melden, dass der Blutzuckerspiegel gerade gemessen wird; und
   einer Meldesteuerungseinrichtung zur Steuerung der Änderung des Meldemodus der Meldeeinrichtung, wenn die Änderung der Reflexionslichtintensität ($\Delta D/\Delta Ax+t$) größer als die erste vorbestimmte Intensität (d) ist und gleich einer zweiten vorbestimmten Intensität (d1) wird, die vor der ersten vorbestimmten Intensität (d) vorliegt.

4. Blutzuckermessinstrument nach Anspruch 3, wobei die Meldesteuerungseinrichtung außerdem meldet, dass mit der Messung begonnen wird, wenn die Färbung der Probe erfasst wird.

5. Blutzuckermessinstrument nach einem der Ansprüche 1 bis 4, wobei die Probe (101) eine Öffnung (102) zum Aufbringen von Blut, einen Wanderabschnitt, zu dem auf die Öffnung aufgebrachtes Blut wandert, und einen Reagensschichtabschnitt (103, 104) hat, der sich durch die Reaktion mit durch den Wanderbereich gegangenem Blut verfärbt, und
die Reflexionslichterfassungseinrichtung (105, 106) von dem Reagensschichtabschnitt stammendes Reflexionslicht erfasst.

6. Blutzuckermessinstrument nach Anspruch 5, wobei der Reagensschichtabschnitt eine Reagensschicht (103), die sich durch die Reaktion mit Blut verfärbt, und eine Filterschicht (104) zum Filtern von Blutzellen des Bluts umfasst.

7. Blutzuckermessinstrument nach Anspruch 5, wobei die ursprüngliche Farbe des Reagensschichtabschnitts (103, 104) im Wesentlichen weiß ist.

8. Blutzuckermessinstrument nach Anspruch 3, wobei die Meldeeinrichtung eine vorbestimmte Markierung mit einer vorbestimmten Geschwindigkeit bewegt und anzeigt und die Meldesteuerungseinrichtung die Bewegungsgeschwindigkeit der vorbestimmten Markierung ändert, wenn die zweite vorbestimmte Intensität (d1) erreicht wird.

9. Verfahren zum Messen des Blutzuckerspiegels von Blut auf Grundlage einer Farbänderung einer Probe (101), in der ein Reagens mit dem Blut reagiert hat, wobei das Verfahren folgende Schritte umfasst:

   Erfassen von Reflexionslicht, indem in Zeitabständen (Ta) Licht auf die Probe (101) fallen gelassen und die Stärke des Reflexionslichts von der Probe (101) erfasst wird;
   Erfassen einer Änderung der Reflexionslichtintensität ($\Delta D/\Delta Ax+t$) in den Zeitabständen (Ta); und
   Berechnen des Blutzuckerspiegels des Bluts auf Grundlage einer Reflexionslichtintensität ($\Delta Ax+t$), die der letzten im Reflexionslichterfassungsschritt erfassten Stärke des Reflexionslichts entspricht, wenn die in dem Änderungserfassungsschritt erfasste Änderung der Reflexionslichtintensität ($\Delta D/\Delta Ax+t$) kleiner oder gleich einer ersten vorbestimmten Intensität (d) geworden ist,

   **dadurch gekennzeichnet, dass**
   der Änderungserfassungsschritt Folgendes beinhaltet: Ermitteln einer Differenz ($\Delta Ax+t - \Delta Ax$) zwischen der Reflexionslichtintensität ($\Delta Ax+t$), die der letzten in dem Reflexionslichterfassungsschritt erfassten Stärke des Reflexionslichts entspricht, und einer Reflexionslichtintensität ($\Delta Ax$), die der Intensität des Reflexionslichts entspricht, die in dem Reflexionslichterfassungsschritt eine vorbestimmte Zeitdauer (t) zuvor erfasst wurde, die länger als die Zeitabstände (Ta) ist, und Bestimmen der Änderung der Reflexionslichtintensität ($\Delta D/\Delta Ax+t$), indem die Differenz ($\Delta Ax+t - \Delta Ax$) durch die vorbestimmte Zeitdauer (t) und die Reflexionslichtintensität ($\Delta Ax+t$) geteilt wird, die der letzten durch den Reflexionslichterfassungsschritt erfassten Stärke des Reflexionslichts entspricht.

10. Verfahren nach Anspruch 9, wobei die Reflexionslichtintensitäten ($\Delta Ax+t$, $\Delta Ax$) in dem Änderungserfassungsschritt auf Grundlage des Absorptionsgrads (A) ermittelt werden, der anhand der Stärke (Aini) des von der Probe (101) stammenden Reflexionslichts vor der Reaktion mit dem Blut und anhand der Stärke (Aaft) des Reflexionslichts nach der Färbung der Probe (101) durch die Reaktion mit dem Blut ermittelt wird.

11. Verfahren zum Messen des Blutzuckerspiegels von Blut nach Anspruch 9 oder 10, mit außerdem den Schritten:

   Melden, dass der Blutzuckerspiegel gerade gemessen wird; und
   Steuern der Änderung des Meldemodus des Meldeschritts, wenn die Änderung der Reflexionslichtintensität ($\Delta D/\Delta Ax+t$) größer als die erste vorbestimmte Intensität (d) ist und gleich einer zweiten vorbestimmten Intensität (d1) wird, die vor der ersten vorbestimmten Intensität (d) vorliegt.

12. Verfahren zum Messen des Blutzuckerspiegels von Blut nach Anspruch 11, wobei der Meldesteuerschritt außerdem meldet, dass mit der Messung begonnen wird, wenn die Färbung der Probe erfasst wird.

13. Verfahren zum Messen des Blutzuckerspiegels von Blut nach einem der Ansprüche 9 bis 12, wobei
   die Probe (101) eine Öffnung (102) zum Aufbringen von Blut, einen Wanderbereich, zu dem auf die Öffnung aufgetragenes Blut wandert, und einen Reagensschichtabschnitt (103, 104) hat, der sich durch die Reaktion mit durch den Wanderbereich gegangenem Blut verfärbt, und
   der Reflexionslichterfassungsschritt von dem Reagensschichtabschnitt stammendes Reflexionslicht erfasst.

14. Verfahren zum Messen des Blutzuckerspiegels von Blut nach Anspruch 13, wobei der Reagensschichtabschnitt eine Reagensschicht (3), die sich durch die Reaktion mit Blut verfärbt, und eine Filterschicht (104) zum Filtern von Blutzellen des Bluts umfasst.

**15.** Verfahren zum Messen des Blutzuckerspiegels von Blut nach Anspruch 13, wobei die ursprüngliche Farbe des Reagensschichtabschnitts (103, 104) im Wesentlichen weiß ist.

**16.** Verfahren zum Messen des Blutzuckerspiegels von Blut nach Anspruch 11, wobei der Meldeschritt eine vorbestimmte Markierung mit einer vorbestimmten Geschwindigkeit bewegt und anzeigt und der Meldesteuerschritt die Bewegungsgeschwindigkeit der vorbestimmten Markierung ändert, wenn die zweite vorbestimmte Intensität (d1) erreicht wird.

**17.** Verfahren zum Messen des Blutzuckerspiegels von Blut nach Anspruch 11 oder 16, wobei der Meldesteuerschritt die zweite vorbestimmte Intensität (d1) entsprechend der gemessenen Glucosekonzentration ändert.

**18.** Verfahren zum Messen des Blutzuckerspiegels von Blut nach einem der Ansprüche 9 bis 17, wobei der Blutzuckerspiegelberechnungsschritt die Umgebungstemperatur misst und den Blutzuckerspiegel entsprechend der Umgebungstemperatur bestimmt.

**Revendications**

**1.** Instrument de mesure du sucre dans le sang destiné à mesurer le taux de sucre dans le sang de sang à partir du changement de couleur d'un spécimen (101) dans lequel un réactant a réagi avec le sang, ledit instrument comprenant :

un moyen de détection de la lumière réfléchie (105, 106) destiné à irradier de la lumière vers ledit spécimen (101) à des intervalles de temps (Ta), et à détecter l'intensité de la lumière réfléchie par le spécimen (101) ;
un moyen de détecter un changement pour détecter un changement dans la quantité de lumière réfléchie ($\Delta D/\Delta Ax+t$) auxdits intervalles de temps (Ta) ; et
un moyen de calculer le sucre dans le sang pour calculer le taux de sucre dans le sang dudit sang sur la base d'une quantité de lumière réfléchie ($\Delta Ax+t$) correspondant à la dernière intensité de la lumière réfléchie détectée par ledit moyen de détecter la lumière réfléchie (105, 106) lorsque le changement de quantité de lumière réfléchie ($\Delta Ax+t$) détecté par ledit moyen de détecter un changement est devenu une première quantité prédéterminée (d) ou moins, **caractérisé en ce que**

ledit moyen de détecter un chargement obtient une différence ($\Delta Ax+t-\Delta Ax$) entre la quantité de lumière réfléchie ($\Delta Ax+t$) correspondant à la dernière intensité de la lumière réfléchie détectée par ledit moyen de détecter la lumière réfléchie (105, 106) et une quantité ($\Delta Ax$) de lumière réfléchie correspondant à l'intensité de la lumière réfléchie détectée par ledit moyen de détecter la lumière réfléchie avant une période de temps (t) prédéterminée qui est plus longue que les intervalles de temps (Ta), et détermine le changement dans la quantité de lumière réfléchie ($\Delta D/\Delta Ax+t$) en divisant ladite différence ($\Delta Ax+t-\Delta Ax$) par la période de temps prédéterminée (t) et la quantité de lumière réfléchie ($\Delta Ax+t$) correspondant à la dernière intensité de la lumière réfléchie détectée par ledit moyen de détecter la lumière réfléchie (105, 106).

**2.** Instrument de mesure du sucre dans le sang selon la revendication 1, où ledit moyen de détecter un changement obtient les quantités de lumière réfléchie ($\Delta Ax+t,\Delta Ax$) sur la base de l'absorbance (A) obtenue à partir de l'intensité (Aini) de la lumière réfléchie par ledit spécimen (101) avant de réagir avec ledit sang, et de l'intensité de la lumière réfléchie (Aaft) après coloration dudit spécimen (101) par la réaction avec ledit sang.

**3.** Instrument de mesure du sucre dans le sang selon la revendication 1 ou 2, comprenant par ailleurs :

un moyen de notification destiné à notifier que le taux de sucre dans le sang est en cours de mesure ; et
un moyen de contrôle de la notification destiné à contrôler le changement de mode de notification dudit moyen de notification lorsque ledit changement de quantité de lumière réfléchie ($\Delta D/\Delta Ax+t$) est plus grand que ladite première quantité prédéterminée (d) et devient une seconde quantité prédéterminée (d1) qui est présente avant ladite première quantité prédéterminée (d).

**4.** Instrument de mesure du sucre dans le sang selon la revendication 3, où ledit moyen de contrôle de la notification notifie par ailleurs que la mesure a commencé à la détection de la coloration dudit spécimen.

**5.** Instrument de mesure du sucre dans le sang selon l'une quelconque des revendications 1 à 4, où ledit spécimen (101) comporte une ouverture (102) destinée à l'application du sang, une portion de circulation vers laquelle se dirige le sang appliqué sur ladite ouverture, et une portion de la couche réactive (103, 104) colorée par la réaction avec le sang qui est passé par ladite zone de circulation, et ledit moyen de détection de la lumière réfléchie (105, 106) détecte la lumière réfléchie par ladite portion de la couche réactive.

**6.** Instrument de mesure du sucre dans le sang selon la revendication 5, où ladite portion de la couche réactive comprend une couche réactive (103) qui est colorée par la réaction avec le sang, et une couche filtre (104) destinée au filtrage des globules sanguins.

**7.** Instrument de mesure du sucre dans le sang selon la revendication 5, où la couleur initiale de ladite portion de la couche réactive (103, 104) est pratiquement blanche.

**8.** Instrument de mesure du sucre dans le sang selon la revendication 3, où ledit moyen de notification bouge et affiche un repère prédéterminé à une vitesse prédéterminée, et ledit moyen de contrôle de notification modifie la vitesse de déplacement dudit repère prédéterminé lorsque ladite seconde quantité prédéterminée (d1) est atteinte.

**9.** Méthode destinée à mesurer le taux de sucre dans le sang de sang sur la base du changement de couleur d'un spécimen (101) dans lequel un réactant a réagi avec le sang, ladite méthode comprenant les étapes de :

détection de la lumière réfléchie par irradiation de lumière vers ledit spécimen (101) à des intervalles de temps (Ta) et détection de l'intensité de la lumière réfléchie par le spécimen (101) ;

calcul du taux de sucre dans le sang dudit sang sur la base d'une quantité de lumière réfléchie ($\Delta Ax+t$) correspondant à la dernière intensité de la lumière réfléchie détectée dans ladite étape de détecter la lumière réfléchie lorsque le changement de quantité de lumière réfléchie détecté (($\Delta D/DAx+t$) dans ladite étape de détecter un changement est devenu une première quantité prédéterminée (d) ou moins, **caractérisée en ce que** ladite étape de détecter un chargement inclut l'obtention d'une différence ($\Delta Ax+t-\Delta Ax$) entre la quantité de lumière réfléchie ($\Delta Ax+t$) correspondant à la dernière intensité de la lumière réfléchie détectée dans ladite étape de détecter la lumière réfléchie et une quantité ($\Delta Ax$) de lumière réfléchie correspondant à l'intensité de la lumière réfléchie détectée dans ladite étape de détecter la lumière réfléchie avant une période de temps (t) prédéterminée qui est plus longue que les intervalles de temps (Ta), et détermination du changement dans la quantité de lumière réfléchie ($\Delta D/\Delta Ax+t$) par division de ladite différence ($\Delta Ax+t - \Delta Ax$) par la période de temps (t) prédéterminée et la quantité de lumière réfléchie ($\Delta Ax+t$) correspondant à la dernière intensité de la lumière réfléchie détectée par ladite étape de détecter la lumière réfléchie.

**10.** Méthode selon la revendication 9, où dans ladite étape de détection du changement, les quantités de lumière réfléchie ($\Delta Ax+t, \Delta Ax$) sont obtenues sur la base de l'absorbance (A) obtenue à partir de l'intensité (Aini) de la lumière réfléchie par ledit spécimen (101) avant de réagir avec ledit sang, et de l'intensité (Aaft) de la lumière réfléchie après coloration dudit spécimen (101) par la réaction avec ledit sang.

**11.** Méthode destinée à mesurer le taux de sucre dans le sang selon la revendication 9 ou 10, comprenant par ailleurs les étapes consistant à :

- notifier que le taux de sucre dans le sang est en cours de mesure ; et
- contrôler pour changer le mode de notification de ladite étape de notification lorsque ledit changement de quantité de lumière réfléchie ($\Delta D/\Delta Ax+t$) devient plus grand que ladite première quantité prédéterminée (d) et devient une seconde quantité prédéterminée (d1) qui est présente avant ladite première quantité prédéterminée (d).

**12.** Méthode destinée à mesurer le taux de sucre dans le sang de sang selon la revendication 11, où ladite étape de contrôle de notification notifie par ailleurs que la mesure a démarré lorsque la coloration dudit spécimen est détectée.

**13.** Méthode destinée à mesurer le taux de sucre dans le sang de sang selon l'une quelconque des revendications 9 à 12, où

ledit spécimen (101) comprend une ouverture (102) destinée à l'application du sang, une zone de circulation vers laquelle le sang appliqué sur ladite ouverture circule, et une portion (103, 104) de la couche réactive colorée par la réaction avec le sang qui est passé par ladite zone de circulation, et

ladite étape de détection de la lumière réfléchie détecte la lumière réfléchie par ladite portion de la couche réactive.

**14.** Méthode destinée à mesurer le taux de sucre dans le sang de sang selon la revendication 13, où ladite portion de la couche réactive comprend une couche réactive (103) qui est colorée par la réaction avec le sang, et une couche filtre (104) destinée au filtrage des globules sanguins.

**15.** Méthode destinée à mesurer le taux de sucre dans le sang de sang selon la revendication 13, où la couleur initiale de ladite portion réactive (103,104) est pratiquement blanche.

**16.** Méthode destinée à mesurer le taux de sucre dans le sang de sang selon la revendication 11, où ladite étape de notification bouge et affiche un repère prédéterminé à une vitesse prédéterminée, et en ce que ladite étape de contrôle de la notification modifie la vitesse de mouvement dudit repère prédéterminé lorsque ladite seconde quantité prédéterminée (d1) est atteinte.

**17.** Méthode destinée à mesurer le taux de sucre dans le sang de sang selon la revendication 11 ou 16, où ladite étape de contrôle de notification modifie ladite seconde quantité prédéterminée (d1) correspondant à la concentration de glucose mesurée.

**18.** Méthode destinée à mesurer le taux de sucre dans le sang de sang selon l'une quelconque des revendications 9 à 17, où ladite étape de calcul du taux de sucre dans le sang mesure la température ambiante et détermine ledit taux de sucre dans le sang correspondant à ladite température ambiante.

# FIG. 1

EP 0 974 303 B2

FIG. 2

# F I G. 3A

# F I G. 3B

# FIG. 4

**F I G.   5**

START

IRRADIATING LIGHT ~ S1

DETERMINE QUANTITY OF REFLECTED LIGHT ~ S2

CHIP MOUNTED? S3
NO
↓ YES

DETERMINE AND STORE QUANTITY A OF REFLECTED LIGHT ~ S4

START OF COLORATION? S5
NO
↓ YES

DETERMINE ABSORBENCY Ax, AND STORE ABSORBENCY C 1 SECOND BEFORE STARTING OF MEASUREMENT ~ S6

1 SECOND ELAPSED? S7
NO
↓ YES

DETERMINE ABSORBENCY Ax S8

DETERMINE CHANGE Δ D IN ABSORBENCY S9

2% OR LESS? S10
NO
↓ YES

DETERMINE BLOOD SUGAR LEVEL ON THE BASIS OF FINAL ABSORBENCY S11

DISPLAY

END S12

FIG. 6

LOW CONCENTRATION
: 7-10 SECONDS

MEDIUM CONCENTRATION
: 10-15 SECONDS

HIGH CONCENTRATION
: 20-40 SECONDS

ABSORBENCY

1180
1080
980
880
780
680
580
480
380
280

TIME

0  5  10  15  20  25  30

◆ LOW CONCENTRATION   ■ MEDIUM CONCENTRATION   ▲ HIGH CONCENTRATION

# F I G. 7

MEASURING TIME (sec)

MEASURED VALUE (mg/dl)

EP 0 974 303 B2

F I G. 8

# FIG. 9

ABSORBENCY X+t SECONDS
AFTER STARTING OF
MEASUREMENT
Ax+t

ABSORBENCY X
SECONDS AFTER
STARTING OF
MEASUREMENT
Ax

START OF ROULETTE DISPLAY
(FIRST DISPLAY SPEED)

END POINT

Tn

ABSORBENCY C
1 SECOND
BEFORE STARTING
OF MEASUREMENT

ROULETTE DISPLAY IS SWITCHED TO
THE SECOND DISPLAY SPEED
BECAUSE THE END POINT (END OF
MEASUREMENT) IS APPROACHING
(ADVANCE NOTICE OF END OF
MEASUREMENT)

T2                                    T3

0  1  2  3  4  5  6  7  8  9  10  11  12

SUCTION OF BLOOD
(START OF COLORING)

EP 0 974 303 B2

F I G. 10

# F I G. 11

```
                    ┌─────────┐
                    │   S5    │
                    └────┬────┘
                         │ YES
                         ▼
        ┌────────────────────────────────┐
        │ START TO DISPLAY IN THE FIRST   │──── S21
        │ DISPLAY MODE                    │
        └────────────────┬───────────────┘
                         │
                         ▼
        ┌────────────────────────────────┐
        │ DETERMINE ABSORBENCY Ax,        │
        │ AND STORE ABSORBENCY C          │──── S22
        │ 1 SECOND BEFORE STARTING OF     │
        │ MEASUREMENT                     │
        └────────────────┬───────────────┘
                         │
      ┌─────────────────►│
      │                  ▼
      │        ╱────────────────────╲  S23
   NO │◄──────◄    1 SECOND          ╲
      │        ╲    ELAPSED?         ╱
      │         ╲──────────┬────────╱
      │                    │ YES
      │                    ▼
      │        ┌────────────────────────────┐
      │        │ DETERMINE ABSORBENCY Ax     │──── S24
      │        └──────────────┬─────────────┘
      │                       │
      │                       ▼
      │        ┌────────────────────────────┐
      │        │ DETERMINE CHANGE ΔD         │──── S25
      │        │ IN ABSORBENCY               │
      │        └──────────────┬─────────────┘
      │                       │
      │                       ▼
      │            ╱────────────────╲  S26
   NO │◄──────────◄     5% OR        ╲
      │            ╲     LESS?       ╱
      │             ╲───────┬───────╱
      │                     │ YES
      │                     ▼
      │        ┌────────────────────────────┐
      │        │ SECOND DISPLAY MODE         │──── S27
      │        └──────────────┬─────────────┘
      │                       │
      │                       ▼
      │            ╱────────────────╲  S28
   NO │◄──────────◄     2% OR        ╲
      │            ╲     LESS?       ╱
      │             ╲───────┬───────╱
      │                     │ YES
      │                     ▼
      │              ┌─────────────┐
      │              │ GO TO S11   │
      │              └─────────────┘
```

# FIG. 12

ABSORBENCY

601

ABSORBENCY C
1 SECOND
BEFORE STARTING
OF MEASUREMENT

602

1 SECOND BEFORE
STARTING OF
MEASUREMENT

SUCTION OF BLOOD
(START OF COLORING)

END POINT AT HIGH
TEMPERATURE

END POINT AT LOW
TEMPERATURE

TIME

EP 0 974 303 B2

FIG. 13